# EUROPEAN PATENT SPECIFICATION

(11) **EP 2 347 266 B1**
(45) Date of publication and mention of the grant of the patent: **17.07.2013**
(21) Application number: 09764179.9
(22) Date of filing: 06.11.2009
(51) Int. Cl.: G01N 33/74

(54) **PROGNOSIS AND RISK ASSESSMENT IN PATIENTS SUFFERING FROM HEART FAILURE BY DETERMINING THE LEVEL OF ADM**
PROGNOSE UND RISIKOBEURTEILUNG BEI AN HERZINSUFFIZIENZ LEIDENDEN PATIENTEN DURCH BESTIMMEN DES ADM-SPIEGELS
PRONOSTIC ET ÉVALUATION DU RISQUE CHEZ DES PATIENTS SOUFFRANT D INSUFFISANCE CARDIAQUE PAR DÉTERMINATION DU TAUX DE ADM

(30) Priority: 11.11.2008 EP 08168816; 12.11.2008 US 113649 P
(43) Date of publication of application: 27.07.2011
(73) Proprietor: B.R.A.H.M.S GmbH, 16761 Hennigsdorf (DE)
(72) Inventor: BERGMANN, Andreas, 12351 Berlin (DE); HARTMANN, Oliver, 10117 Berlin (DE)
(74) Representative: Kilger, Ute
(86) International application number: PCT/EP2009/008056
(87) International publication number: WO 2010/054810

(56) References cited:
- VON HAEHLING S ET AL: "Mid-regional pro-adrenomedullin as a novel and prognostic marker in chronic heart failure (CHF)" EUROPEAN HEART JOURNAL, vol. 27, no. Suppl. 1, August 2006 (2006-08), page 16, XP002569337 & WORLD CONGRESS OF CARDIOLOGY; BARCELONA, SPAIN; SEPTEMBER 02 -06, 2006 ISSN: 0195-668X
- DATABASE BIOSIS [Online] BIOSCIENCES INFORMATION SERVICE, PHILADELPHIA, PA, US; October 2006 (2006-10), NISHKIMI TOSHIO ET AL: "Plasma adrenomedullin level is a long-term prognostic marker in patients with chronic stable cardiovascular disease" XP002569338 Database accession no. PREV200700123037 & CIRCULATION, vol. 114, no. 18, Suppl. S, October 2006 (2006-10), page 529, 79TH ANNUAL SCIENTIFIC SESSION OF THE AMERICAN-HEART-ASSOCIATION; CHICAGO, IL, USA; NOVEMBER 12 -15, 2006 ISSN: 0009-7322
- COLETTA A P ET AL: "Clinical trials update from the heart failure society of America and the American heart association meetings in 2008: SADHART-CHF, COMPARE, MOMENTUM, thyroid hormone analogue study, HF-ACTION, I-PRESERVE, [beta]-interferon study, BACH, and ATHENA" EUROPEAN JOURNAL OF HEART FAILURE 2009 OXFORD UNIVERSITY PRESS GBR, vol. 11, no. 2, 2009, pages 214-219, XP002569339
- POTOCKI M ET AL: "Midregional pro-Adrenomedullin in addition to b-type natriuretic peptides in the risk stratification of patients with acute dyspnea: An observational study" CRITICAL CARE 20090723 BIOMED CENTRAL LTD. GBR, vol. 13, no. 4, 122, 23 July 2009 (2009-07-23), XP002569340
- GEGENHUBER ET AL: "Comparative Evaluation of B-Type Natriuretic Peptide, Mid-Regional Pro-A-type Natriuretic Peptide, Mid-Regional Pro-Adrenomedullin, and Copeptin to Predict 1-Year Mortality in Patients With Acute Destabilized Heart Failure" JOURNAL OF CARDIAL FAILURE, CHURCHILL LIVINGSTONE, NAPERVILLE, IL, US, vol. 13, no. 1, 3 March 2007 (2007-03-03), pages 42-49, XP005910325 ISSN: 1071-9164
- WRITING GROUP MEMBERS ET AL: "National Academy of Clinical Biochemistry Laboratory Medicine Practice Guidelines: Clinical Utilization of Cardiac Biomarker Testing in Heart Failure" CLINICAL BIOCHEMISTRY, ELSEVIER INC, US, CA, vol. 41, no. 4-5, 1 March 2008 (2008-03-01), pages 210-221, XP022701481 ISSN: 0009-9120 [retrieved on 2008-02-15]
- KHAN ET AL: "Prognostic Value of Midregional Pro-Adrenomedullin in Patients With Acute Myocardial Infarction" JOURNAL OF THE AMERICAN COLLEGE OF CARDIOLOGY, ELSEVIER, NEW YORK, NY, US, vol. 49, no. 14, 3 April 2007 (2007-04-03) , pages 1525-1532, XP022016280 ISSN: 0735-1097
- DATABASE BIOSIS [Online] BIOSCIENCES INFORMATION SERVICE, PHILADELPHIA, PA, US; October 2008 (2008-10), WILD PHILIPP S ET AL: "Midregional Pro-Adrenomedullin for Risk Stratification in Coronary Artery Disease - Results from the AtheroGene Study" XP002569341 Database accession no. PREV200900198016 & CIRCULATION, vol. 118, no. 18, Suppl. 2, October 2008 (2008-10), page S584, 81ST ANNUAL SCIENTIFIC SESSION OF THE AMERICAN-HEART-ASSOCIATION; NEW ORLEANS, LA, USA; NOVEMBER 08 -12, 2008 ISSN: 0009-7322
- CHRIST M ET AL: "Multimarker strategy for risk prediction in patients presenting with acute dyspnea to the emergency department" INTERNATIONAL JOURNAL OF CARDIOLOGY, ELSEVIER SCIENCE PUBLISHERS, AMSTERDAM, NL, vol. 126, no. 1, 7 May 2008 (2008-05-07), pages 73-78, XP022611880 ISSN: 0167-5273 [retrieved on 2008-04-15]
- MORGENTHALER ET AL.: CLINICAL CHEMISTRY, vol. 51, no. 10, 2005, pages 1823-1829,
- ANON: 'Peptides', [Online] 2006, Website Retrieved from the Internet: <URL:www.anaspec.com> [retrieved on 2012-06-10]

### SEQUENCE LISTING

<110> B.R.A.H.M.S AG
<120> Prognosis and risk assessment in patients suffering from heart failure by determining the level of ADM and BNP
<130> B60408PCT
<150> 08168816.0
   <151> 2008-11-11
<160> 9
<170> PatentIn version 3.3
<210> 1
   <211> 185
   <212> PRT
   <213> Homo sapiens
<400> 1
   Met Lys Leu Val Ser Val Ala Leu Met Tyr Leu Gly Ser Leu Ala Phe
   1 5 10 15
   Leu Gly Ala Asp Thr Ala Arg Leu Asp Val Ala Ser Glu Phe Arg Lys
   20 25 30
   Lys Trp Asn Lys Trp Ala Leu Ser Arg Gly Lys Arg Glu Leu Arg Met
   35 40 45
   Ser Ser Ser Tyr Pro Thr Gly Leu Ala Asp Val Lys Ala Gly Pro Ala
   50 55 60
   Gln Thr Leu Ile Arg Pro Gln Asp Met Lys Gly Ala Ser Arg Ser Pro
   65 70 75 80
   Glu Asp Ser Ser Pro Asp Ala Ala Arg Ile Arg Val Lys Arg Tyr Arg
   85 90 95
   Gln Ser Met Asn Asn Phe Gln Gly Leu Arg Ser Phe Gly Cys Arg Phe
   100 105 110
   Gly Thr Cys Thr Val Gln Lys Leu Ala His Gln Ile Tyr Gln Phe Thr
   115 120 125
   Asp Lys Asp Lys Asp Asn Val Ala Pro Arg Ser Lys Ile Ser Pro Gln
   130 135 140
   Gly Tyr Gly Arg Arg Arg Arg Arg Ser Leu Pro Glu Ala Gly Pro Gly
   145 150 155 160
   Arg Thr Leu Val Ser Ser Lys Pro Gln Ala His Gly Ala Pro Ala Pro
   165 170 175
   Pro Ser Gly Ser Ala Pro His Phe Leu
   180 185
<210> 2
   <211> 164
   <212> PRT
   <213> Homo sapiens
<400> 2
   Ala Arg Leu Asp Val Ala Ser Glu Phe Arg Lys Lys Trp Asn Lys Trp
   1               5                   10                  15
   Ala Leu Ser Arg Gly Lys Arg Glu Leu Arg Met Ser Ser Ser Tyr Pro
               20                  25                  30
   Thr Gly Leu Ala Asp Val Lys Ala Gly Pro Ala Gln Thr Leu Ile Arg
           35                  40                  45
   Pro Gln Asp Met Lys Gly Ala Ser Arg Ser Pro Glu Asp Ser Ser Pro
       50                  55                  60
   Asp Ala Ala Arg Ile Arg Val Lys Arg Tyr Arg Gln Ser Met Asn Asn
   65                  70                  75                  80
   Phe Gln Gly Leu Arg Ser Phe Gly Cys Arg Phe Gly Thr Cys Thr Val
                   85                  90                  95
   Gln Lys Leu Ala His Gln Ile Tyr Gln Phe Thr Asp Lys Asp Lys Asp
               100                 105                 110
   Asn Val Ala Pro Arg Ser Lys Ile Ser Pro Gln Gly Tyr Gly Arg Arg
           115                 120                 125
   Arg Arg Arg Ser Leu Pro Glu Ala Gly Pro Gly Arg Thr Leu Val Ser
       130                 135                 140
   Ser Lys Pro Gln Ala His Gly Ala Pro Ala Pro Pro Ser Gly Ser Ala
   145                 150                 155                 160
   Pro His Phe Leu
<210> 3
   <211> 20
   <212> PRT
   <213> Homo sapiens
<400> 3
   Ala Arg Leu Asp Val Ala Ser Glu Phe Arg Lys Lys Trp Asn Lys Trp
   1               5                   10                  15
   Ala Leu Ser Arg
               20
<210> 4
   <211> 40
   <212> PRT
   <213> Homo sapiens
<400> 4
<210> 5
   <211> 52
   <212> PRT
   <213> Homo sapiens
<400> 5
   Tyr Arg Gln Ser Met Asn Asn Phe Gln Gly Leu Arg Ser Phe Gly Cys
   1 5 10 15
   Arg Phe Gly Thr Cys Thr Val Gln Lys Leu Ala His Gln Ile Tyr Gln
   20 25 30
   Phe Thr Asp Lys Asp Lys Asp Asn Val Ala Pro Arg Ser Lys Ile Ser
   35 40 45
   Pro Gln Gly Tyr
   50
<210> 6
   <211> 134
   <212> PRT
   <213> Homo sapiens
<400> 6
   Met Asp Pro Gln Thr Ala Pro Ser Arg Ala Leu Leu Leu Leu Leu Phe
   1 5 10 15
   Leu His Leu Ala Phe Leu Gly Gly Arg Ser His Pro Leu Gly Ser Pro
   20 25 30
   Gly Ser Ala Ser Asp Leu Glu Thr Ser Gly Leu Gln Glu Gln Arg Asn
   35 40 45
   His Leu Gln Gly Lys Leu Ser Glu Leu Gln Val Glu Gln Thr Ser Leu
   50 55 60
   Glu Pro Leu Gln Glu Ser Pro Arg Pro Thr Gly Val Trp Lys Ser Arg
   65 70 75 80
   Glu Val Ala Thr Glu Gly Ile Arg Gly His Arg Lys Met Val Leu Tyr
                   85                  90                  95
   Thr Leu Arg Ala Pro Arg Ser Pro Lys Met Val Gln Gly Ser Gly Cys
               100                 105                 110
   Phe Gly Arg Lys Met Asp Arg Ile Ser Ser Ser Ser Gly Leu Gly Cys
           115                 120                 125
   Lys Val Leu Arg Arg His
       130
<210> 7
   <211> 108
   <212> PRT
   <213> Homo sapiens
<400> 7
   His Pro Leu Gly Ser Pro Gly Ser Ala Ser Asp Leu Glu Thr Ser Gly
   1               5                   10                  15
   Leu Gln Glu Gln Arg Asn His Leu Gln Gly Lys Leu Ser Glu Leu Gln
               20                  25                  30
   Val Glu Gln Thr Ser Leu Glu Pro Leu Gln Glu Ser Pro Arg Pro Thr
           35                  40                  45
   Gly Val Trp Lys Ser Arg Glu Val Ala Thr Glu Gly Ile Arg Gly His
       50                  55                  60
   Arg Lys Met Val Leu Tyr Thr Leu Arg Ala Pro Arg Ser Pro Lys Met
   65                  70                  75                  80
   Val Gln Gly Ser Gly Cys Phe Gly Arg Lys Met Asp Arg Ile Ser Ser
                   85                  90                  95
   Ser Ser Gly Leu Gly Cys Lys Val Leu Arg Arg His
               100                 105
<210> 8
   <211> 76
   <212> PRT
   <213> Homo sapiens
<400> 8
   His Pro Leu Gly Ser Pro Gly Ser Ala Ser Asp Leu Glu Thr Ser Gly
   1               5                   10                  15
   Leu Gln Glu Gln Arg Asn His Leu Gln Gly Lys Leu Ser Glu Leu Gln
               20                  25                  30
   Val Glu Gln Thr Ser Leu Glu Pro Leu Gln Glu Ser Pro Arg Pro Thr
           35                  40                  45
   Gly Val Trp Lys Ser Arg Glu Val Ala Thr Glu Gly Ile Arg Gly His
       50                  55                  60
   Arg Lys Met Val Leu Tyr Thr Leu Arg Ala Pro Arg
   65                  70                  75
<210> 9
   <211> 32
   <212> PRT
   <213> Homo sapiens
<400> 9
   Ser Pro Lys Met Val Gln Gly Ser Gly Cys Phe Gly Arg Lys Met Asp
   1               5                   10                  15
   Arg Ile Ser Ser Ser Ser Gly Leu Gly Cys Lys Val Leu Arg Arg His
               20                  25                  30

## Description

### Field of the invention

The present invention is in the field of clinical diagnostics. Particularly the present invention relates to short-term outcome prognosis in patients suffering from heart failure by determination of the level of the marker peptide ADM.

### Background of the invention

Heart failure is a cardiac condition that occurs when a problem with the structure or function of the heart impairs its ability to supply sufficient blood flow to meet the body's needs. It can cause a large variety of symptoms, particularly shortness of breath and ankle swelling, but some patients can be completely symptom free. Heart failure is often undiagnosed due to a lack of a universally agreed definition and challenges in definitive diagnosis, particularly in early stage. With appropriate therapy, heart failure can be managed in the majority of patients, but it is a potentially life threatening condition, and progressive disease is associated with an annual mortality of 10%. It is the leading cause of hospitalization in people older than 65.

In this regard, Von Haehling *et al.* disclosed MR-pro-ADM and NT-proBNP as independent prognostic markers for congestive heart failure in terms of long-term prognosis *("*Mid-regional pro-adrenomedullin as a novel and prognostic marker in chronic heart failure (CHF), European Heart Journal, Vo. 27, No. Suppl. 1, August 2006, page 16*, XP0025b9337; also see* World Congress of cardiology; Barcelona, Spain, on September 2, 2006, ISSN: 0195-668X*).*

However, the inventors surprisingly found that the determination of the level adrenomedullin (ADM) or its fragments thereof or its precursors or its fragments thereof can be reliably correlated with the short-term prognosis up to 90 days of an outcome for patients suffering from heart failure and/ or shortness of breath, and in addition can be reliably correlated with the stratification into risk groups of respective patients.

### Description of the invention

A subject of the present invention is the provision of a method for prognosis of an outcome after a time period of up to 90 days of a patient suffering from heart failure and/or shortness of breath, comprising the steps of:
a. providing a sample obtained from said patient,
b. determining the level of adrenomedullin (ADM) or fragments thereof or its precursor or fragments thereof in said sample, wherein the fragments are of at least six amino acids in length
c. correlating said level with said prognosis.

In another embodiment the present invention provides for a method for prognosis of an outcome after a time period of up to 90 days of a patient suffering from heart failure and/or shortness of breath, wherein the level of troponin is determined in addition to adrenomedullin (ADM) or fragments thereof or its precursor or fragments thereof as detailed above under steps a) to c).

In another embodiment of the invention said outcome regards survival and/or a functional outcome.

In another embodiment the present invention provides for a method for prognosis of an outcome after a time period of up to 90 days of a patient suffering from heart failure and/or shortness of breath according to the steps a) to c), wherein the outcome after 3 days, 5 days, 10 days, 14 days, 20 days, 3 weeks, 4 weeks, 30 days, preferably 30 days is predicted.

In another embodiment the present invention provides for a method for the stratification of a patient into risk groups, wherein said patient is suffering from heart failure and/or shortness of breath and said method comprises the steps a) to c) as detailed above.

In another embodiment the present invention provides for a method for prognosis of an outcome after a time period of up to 90 days of a patient suffering from heart failure and/or shortness of breath according to the steps a) to c), wherein adrenomedullin (ADM) or fragments thereof or its precursor or fragments thereof is MR-proADM.

In another embodiment of the invention said outcome regards survival.

In another embodiment of the invention said functional outcome is determined as ranking or the degree of severity of the outcome.

In another embodiment the present invention provides for a method for prognosis of an outcome after a time period of up to 90 days of a patient suffering from heart failure and/or shortness of breath according to the steps a) to c), wherein additionally at least one clinical parameter is determined selected from the group comprising: age, gender, systolic blood pressure, diastolic blood pressure, antihypertensive treatment, body mass index, heart rate, temperature, presence of diabetes mellitus, current smoking habits.

Also disclosed is a method for prognosis of an outcome after a time period of up to 90 days of a patient suffering from heart failure and/or shortness of breath according to the steps a) to c), wherein additionally at least one other laboratory parameter is determined selected from the group comprising troponin, myeloperoxidase, CRP, neopterin, GDF-15, ST2, cystatin-C, as well as the following peptides in form of their mature peptides, precursors, pro-hormones and associated prohormone fragments: atrial natriuretic peptide, endothelins, vasopressin.

In another embodiment the present invention provides for the use of the methods as detailed above for monitoring of the therapy in a patient suffering from heart failure and/or shortness of breath.

Heart failure herein preferably relates to congestive heart failure (CHF). The heart failure may preferably be an acute heart failure (AHF).

It is preferred in this particular embodiment that the short time outcome, i.e. the outcome within or after 45 days, 40 days, 35 days, 30 days, 25 days, 20 days, 15 days, 10 days or 5 days, preferably after 30 days is predicted. In another preferred embodiment of the invention the outcome up to 90 days is predicted by short-term prognosis pursuant to the invention. The invention also relates to the use of the described methods for short-term prognosis of an outcome up to 90 days of a patient suffering from heart failure and/or shortness of breath. Furthermore, the invention relates to the use of the methods for monitoring of the therapy in a patient suffering from heart failure and/or shortness of breath.

The levels of the markers as obtained by the methods or the use of the methods according to the present invention may be analyzed in a number of fashions well known to a person skilled in the art. For example, each assay result obtained may be compared to a "normal" value, or a value indicating a particular disease or outcome. A particular prognosis may depend upon the comparison of each assay result to such a value, which may be referred to as a prognostic "threshold". In certain embodiments, assays for one or more prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background.

The sensitivity and specificity of a prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples obtained from patient might be ranked according to degree (e.g. 1=low, 2=normal, and 3=high). This ranking can be correlated to results in the "normal" population, and a ROC curve created. These methods are well known in the art. See, *e.g.,* Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, particular thresholds for one or more markers in a panel are not relied upon to determine if a profile of marker levels obtained from a subject are indicative of a particular prognosis. Rather, the present invention may utilize an evaluation of a marker panel "profile" as a unitary whole. A particular "fingerprint" pattern of changes in such a panel of markers may, in effect, act as a specific prognostic indicator. As discussed herein, that pattern of changes may be obtained from a single sample, or from temporal changes in one or more members of the panel (or a panel response value). A panel herein refers to a set of markers.

As described herein after, a panel response value is preferably determined by plotting ROC curves for the sensitivity (i.e. true positives) of a particular panel of markers versus 1-(specificity) (i.e. false positives) for the panel at various cut-offs. In these methods, a profile of marker measurements from a subject is considered together to provide a global probability (expressed either as a numeric score or as a percentage risk) of prognosis. In such embodiments, an increase in a certain subset of markers may be sufficient to indicate a particular prognosis in a sample obtained from one patient, while an increase in a different subset of markers may be sufficient to indicate the same or a different prognosis in a sample obtained from another patient. Weighting factors may also be applied to one or more markers in a panel, for example, when a marker is of particularly high utility in identifying a particular prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/5% of a given measurement.

The skilled artisan will understand that associating a prognostic indicator, with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.,* Dowdy and Warden, Statistics for Research, John Wiley & Sons, New York, 1983*.* Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

In yet other embodiments, multiple determinations of prognostic markers can be made, and a temporal change in the marker can be used to determine a prognosis. For example, a marker concentration in a sample obtained from subject may be determined at an initial time, and again at a second time from a second sample obtained from subject. In such embodiments, an increase in the marker from the initial time to the second time may be indicative of a particular prognosis. Likewise, a decrease in the marker from the initial time to the second time may be indicative of a particular prognosis.

The term "sample" as used herein refers to a sample of bodily fluid obtained from a subject or patient for the purpose of prognosis, or evaluation of a subject of interest, such as a patient. Preferred test samples obtained from subject/ patient include blood, serum, plasma, cerebrospinal fluid, urine, saliva, sputum, and pleural effusions. In addition, one of skill in the art would realize that some test samples obtained from subject/ patient would be more readily analyzed following a fractionation or purification procedure, for example, separation of whole blood into serum or plasma components.

Thus, in a preferred embodiment of the invention the sample obtained from subject/ patient is selected from the group comprising a blood sample, a serum sample, a plasma sample, a cerebrospinal fluid sample, a saliva sample and a urine sample or an extract of any of the aforementioned samples. Preferably, the sample obtained from subject/ patient is a blood sample, most preferably a serum sample or a plasma sample.

The term "patient" as used herein refers to a living human or non-human organism that is receiving medical care or that should receive medical care due to a disease. This includes persons with no defined illness who are being investigated for signs of pathology. Thus, the methods and assays described herein are applicable *in vitro* to both human and veterinary disease.

The term "correlating," as used herein in reference to the use of prognostic markers, refers to comparing the presence or amount of the marker(s) in samples obtained from a patient to its presence or amount in persons known to suffer from, or known to be at risk of, a given condition; or in persons known to be free of a given condition. As discussed above, a marker level in a sample obtained from patient can be compared to a level known to be associated with a specific diagnosis. In preferred embodiments, a panel of marker levels is correlated to a global probability or a particular outcome.

The abbreviation ADM in the context of the present invention relates to adrenomedullin or fragments thereof or precursors or fragments thereof. A preferred fragment of a precursor of ADM is mid-regional proADM (MR-proADM). MR-proADM₂₄₋₇₁ (or MR-preproADM₄₅₋₉₂) is a particularly preferred marker peptide in the context of the present invention.

"Fragments" of the marker peptides relate to fragments of at least 12 amino acids in length, preferably at least six amino acid residues in length.

The term "level" in the context of the present invention relates to the concentration (preferably expressed as weight/volume; w/v) of marker peptides in a sample taken from a patient.

The term "outcome" herein relates for instance to the survival of the patient after a defined time, e.g. after 3 days, 5 days, 10 days, 14 days, 20 days, 3 weeks, 4 weeks, 30 days, 45 days, 60 days, 90 days, , preferably 30 days.

The term "functional outcome" in the context of the present invention relates to the degree of severity of the disease, i.e. the state of health the patient after a defined time, e.g. 3 days, 5 days, 10 days, 14 days, 20 days, 3 weeks, 4 weeks, 30 days, 45 days, 60 days, 90 days, preferably 30 days.

Determining (or measuring or detecting) the level of a marker peptide herein is performed using a detection method and/or a diagnostic assay as explained below.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present invention, "capture molecules" are molecules which may be used to bind target molecules or molecules of interest, i.e. analytes (i.e. in the context of the present invention the cardiovascular peptide(s)), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a RNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof, preferably at least six amino acids thereof.

The preferred detection methods comprise immunoassays in various formats such as for instance radioimmunoassay (RIA), chemiluminescence- and fluorescence- immunoassays, Enzyme-linked immunoassays (ELISA), Luminex-based bead arrays, protein microarray assays, and rapid test formats such as for instance immunochromatographic strip tests.

The assays can be homogenous or heterogeneous assays, competitive and non-competitive sandwich assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person. *(*The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10*. PMID: 16376134).*

In a particularly preferred embodiment the assay comprises two capture molecules, preferably antibodies which are both present as dispersions in a liquid reaction mixture, wherein a first labeling component is attached to the first capture molecule, wherein said first labeling component is part of a labeling system based on fluorescence- or chemiluminescence-quenching or amplification, and a second labeling component of said marking system is attached to the second capture molecule, so that upon binding of both capture molecules to the analyte a measurable signal is generated that allows for the detection of the formed sandwich complexes in the solution comprising the sample.

Even more preferred, said labeling system comprises rare earth cryptates or rare earth chelates in combination with a fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, including citations on pages 551-562. Preferred chemiluminescent dyes are acridiniumesters.

The levels, i.e. the concentrations, of the one or more marker peptides (or fragments thereof or precursors or fragments thereof) in the sample obtained from the patient are attributed to the short-term prognosis of an outcome for the patient. For instance, concentrations of the marker peptide above a certain threshold value are indicative for an adverse outcome or an elevated risk for the patient. Such threshold values are preferably in the range of from about 0.5 to 5.0 pmol/l, more preferably 1.0 to 2.5 pmol/l, most preferably 1.985 nmol/l for MR-proADM.

In another embodiment of the invention, the risk and/or outcome for a patient is determined by relating the patient's individual level of marker peptide to certain percentiles (e.g. 97.5^{th} percentile) of a healthy population.

Kaplan-Meier estimators may be used for the assessment or prediction of the outcome or risk (e.g. morbidity) of a patient.

The teaching of the present invention is derived from a comparative study among adrenomedullin (ADM) or fragments thereof or its precursor or fragments thereof, and troponin, and the brain natriuretic peptide (BNP) or fragments thereof or its precursor or fragments thereof regarding its prognostic values in shortness of breath (SoB) and acute heart failure (AHF) patients.

### Sequences

The amino acid sequence of the precursor peptide of Adrenomedulin (pre-pro-Adrenomedullin) is given in SEQ ID NO:1. Pro-Adrenomedullin relates to amino acid residues 22 to 185 of the sequence of pre-pro-Adrenomedullin. The amino acid sequence of pro-Adrenomedullin (pro-ADM) is given in SEQ ID NO:2. The pro-ADM N-terminal 20 peptide (PAMP) relates to amino acid residues 22-41 of pre-proADM. The amino acid sequence of PAMP is given in SEQ ID NO:3. MR-pro-Adrenomedullin (MR-pro-ADM) relates to amino acid residues 45-92 of pre-pro-ADM. The amino acid sequence of MR-pro-ADM is provided in SEQ ID NO:4. The amino acid sequence of mature Adrenomedullin (ADM) is given in SEQ ID NO:5.

The sequence of the 134 amino acid precursor peptide of brain natriuretic peptide (pre-pro-BNP) is given in SEQ ID NO:6 . Pro-BNP relates to amino acid residues 27 to 134 of pro-pro-BNP. The sequence of pro-BNP is shown in SEQ ID NO:7. Pro-BNP is cleaved into N-terminal pro-BNP (NT-pro-BNP) and mature BNP. NT-pro-BNP comprises the amino acid residues 27 to 102 and its sequence is shown in SEQ ID NO:8. The SEQ ID NO:9 shows the sequence of BNP comprising the amino acid residues 103 to 134 of the pre-pro-BNP peptide.
SEQ ID NO:1 (amino acid sequence of pre-pro-ADM):
SEQ ID NO:2 (amino acid sequence of pro-ADM):
SEQ ID NO:3 (amino acid sequence of pro-ADM N20):
   1 ARLDVASEFR KKWNKWALSR
SEQ ID NO:4 (amino acid sequence of MR-pro-ADM):
   1 ELRMSSSYPT GLADVKAGPA QTLIRPQDMK GASRSPEDSS PDAARIRV
SEQ ID NO:5 (amino acid sequence of ADM):
SEQ ID NO:6 (amino acid sequence of pre-pro-BNP) :
SEQ ID NO:7 (amino acid sequence of pro-BNP) :
SEQ ID NO:8 (amino acid sequence of NT-pro-BNP):
SEQ ID NO:9 (amino acid sequence of BNP) :
   1 SPKMVQGSGC FGRKMDRISS SSGLGCKVLR RH

### Description of drawings

Fig. 1: Survival rates plotted for the four quartiles of MR-proADM levels in AHF patients.
Fig. 2: Survival rates plotted for the four quartiles of MR-proADM levels in AHF patients, quartiles 1 to 3 have been combined.
Fig. 3: Survival rates plotted for the four quartiles ofBNP levels in AHF patients.
Fig. 4: Survival rates plotted for the four quartiles of BNP levels in AHF patients, quartiles 1 to 3 have been combined.
Fig. 5: Survival rates plotted for the four quartiles of NT-proBNP levels in AHF patients.
Fig. 6: Survival rates plotted for the four quartiles of NT-proBNP levels in AHF patients, quartiles 1 to 3 have been combined.
Fig. 7: Area under the curve (AUC) from ROC plots for the markers BNP, NT-proBNP and MR-proADM on different days.
Fig. 8: ROC plot for the markers BNP, NT-proBNP and MR-proADM.
Fig. 9: Survival (cumulative survival depending on the day) in patients with and without AHF plotted for MR-proADM above and below the threshold of 1.985 pmol/L.
Fig. 10: Summary of patients.

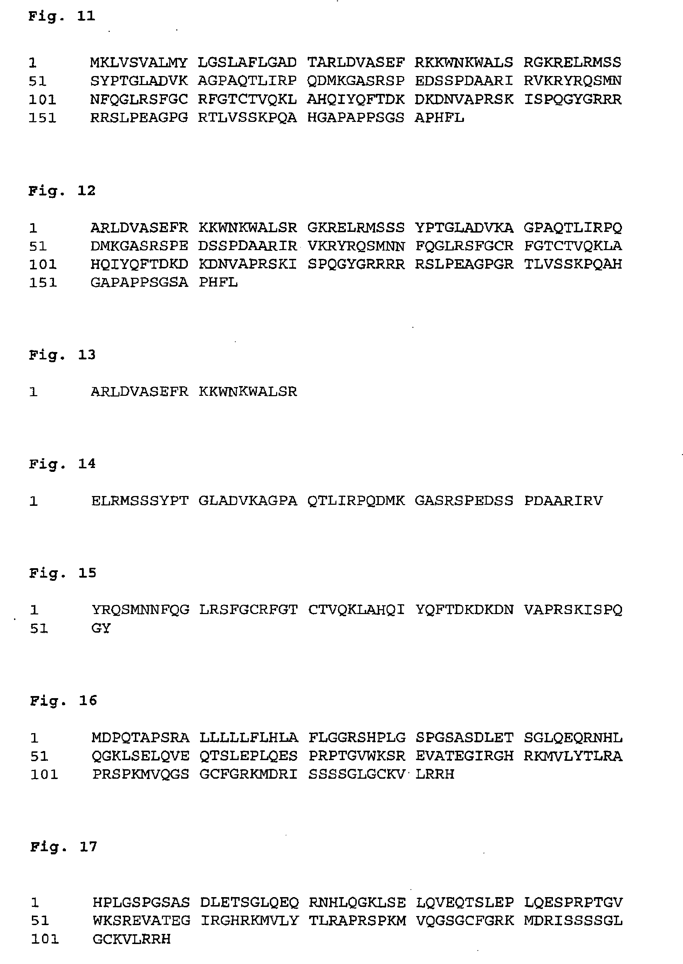

Fig. 11: SEQ ID NO:1 (amino acid sequence of pre-pro-ADM)
Fig. 12: SEQ ID NO:2 (amino acid sequence of pro-ADM)
Fig. 13: SEQ ID NO:3 (amino acid sequence of pro-ADM N20)
Fig. 14: SEQ ID NO:4 (amino acid sequence of MR-proADM)
Fig. 15: SEQ ID NO:5 (amino acid sequence of ADM)
Fig. 16: SEQ ID NO:6 (amino acid sequence of pre-pro-BNP)

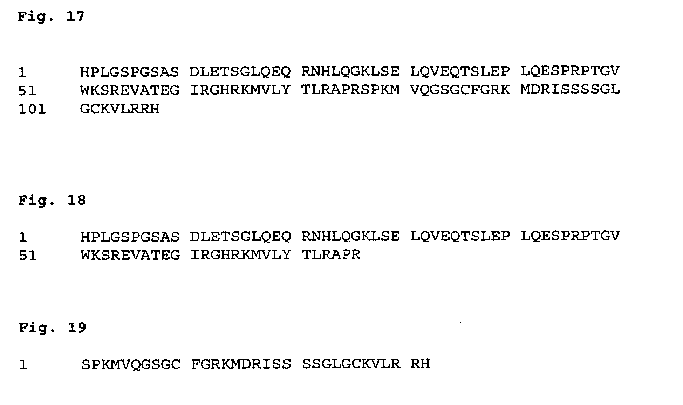

Fig. 17: SEQ ID NO:7 (amino acid sequence of pro-BNP)
Fig. 18: SEQ ID NO:8 (amino acid sequence of NT-pro-BNP)
Fig. 19: SEQ ID NO:9 (amino acid sequence of BNP)

### Examples

### Example 1: Clinical study: 15 Enrolling Centers - recruiting 1641 patients with shortness of breath (SoB) and acute heart failure (AHF)

**Table 1: Summary of patients**

| Characteristics | | **B**reathing **N**ot **P**roperly | **BACH** |
|---|---|---|---|
| **TOTAL ENROLLMENT** | | **1,586** | **1,641** |
| **Age** | | **64 +/-17** | **64 +/- 17** |
| **Sex Male (%)** | | **56** | **52** |
| **Sex Female (%)** | | **44** | **48** |
| **History (%)** | | | |
| | **CHF** | **33** | **36** |
| | **AMI** | **27** | **19** |
| | **COPD** | **41** | **30** |
| | **Diabetes** | **25** | **29** |

**Table 2: Additional baseline data of patients**

| **Variable** | **Non-AHF** | | **AHF** | | **p** |
|---|---|---|---|---|---|
| | **Mean** | **SD** | **Mean** | **SD** | |
| Heart Rate (bpm) | 93 | 22 | 89 | 25 | 0.005 |
| Temperature (C) | 36.8 | 0.7 | 36.7 | 0.7 | 0.039 |
| Systolic BP (mmHg) | 140 | 27 | 143 | 32 | 0.027 |
| Diastolic BP (mmHg) | 80 | 16 | 83 | 19 | <0.001 |
| BMI (kg/m2) | 30 | 9 | 29 | 8 | 0.035 |

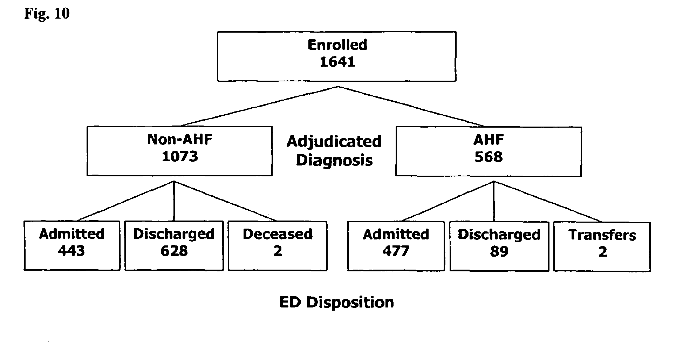

Also see Fig. 10 for summary of patients.

### Study particulars:

- Patients included who presented to emergency department (ED) with SoB not from trauma, or obvious myocardial infarction (MI), and not on dialysis.
- After consenting, MD assessment of probability of heart failure and/or pneumonia.
- Two independent cardiologists agreed on final diagnosis following discharge.
- Follow-up for up to 90 days for survival; Outcome "All cause mortality within 90 days".

### Survival in AHF - Results of Cox regression with continuous predictors:

| | | | |
|---|---|---|---|
| Diagnostic Accuracy: | MR-proADM 73.5% | vs BNP 60.8% | (p<0.001) |
| | | vs NT-proBNP 63.6% | (p<0.001) |

**Table 3: MR-proADM is superior to BNP and NT-proBNP for predicting 90-day mortality (Cox regression).**

| **Predictor (univariate)** | **Chi² Statistic** | **p** | **c index** |
|---|---|---|---|
| log MR-proADM | 31.0 | <0.001 | 0.669 |
| log BNP | 7.1 | 0.008 | 0.596 |
| log NT-proBNP | 17.1 | <0.001 | 0.654 |

**Table 4: MR-proADM adds significantly to BNP or NT-proBNP, however neither BNP nor NT-proBNP add to MR-proANP**

| | **Chi² Statistic** | **p** |
|---|---|---|
| adding MR-proADM to BNP | 23.9 | <0.0001 |
| adding MR-proADM to NT-proBNP | 15.3 | <0.0001 |
| adding BNP to MR-proADM | 0.0 | 0.906 |
| adding NT-proBNP to MR-proADM | 1.1 | 0.291 |

### Survival in AHF - Results of multivariable Cox regression:

**Table 5: MR-proADM is more important than BNP in survival models using Cox Regression.**

| **Predictor (multivariable)** | **HR** | **95% CI** | **p** |
|---|---|---|---|
| log MR-proADM | 14.0 | 5.0-39.4 | <0.001 |
| log BNP | 1.0 | 0.5-2.0 | 0.906 |

**Table 6: MR-proADM is more important than NT-proBNP in survival models using Cox Regression.**

| **Predictor (multivariable)** | **HR** | **95% CI** | **p** |
|---|---|---|---|
| log MR-proADM | 10.4 | 3.3-32.7 | <0.001 |
| log NT-proBNP | 1.4 | 0.7-2.6 | 0.295 |

### Survival in AHF - Cox Models with Troponin Elevation

Troponin values were available in 511 of 568 HF patients in 107 (20.9%) patients they were elevated.

**Table 7: In models with 3 markers, Troponin & MR-proADM provide prognostic utility, but BNP does not.**

| **Predictor (multivariable)** | **HR** | **95% CI** | **p** |
|---|---|---|---|
| log MR-proADM | 8.5 | 2.7-26.5 | <0.001 |
| log BNP | 0.9 | 0.5-1.9 | 0.812 |
| Elevated Tn | 2.6 | 1.5-4.5 | <0.001 |

**Table 8: In models with 3 markers, Troponin & MR-proADM provide prognostic utility, but NT-proBNP does not.**

| **Predictor (multivariable)** | **HR** | **95% CI** | **p** |
|---|---|---|---|
| log MR-proADM | 7.5 | 2.1-26.4 | <0.001 |
| log NT-proBNP | 1.1 | 0.6-2.2 | 0.295 |
| Elevated Tn | 2.6 | 1.5-4.4 | <0.001 |

Clinical lab Troponin values (TnI or TnT) were judged as elevated if above the local normal range.

### Survival in AHF - MR-proADM Quartiles

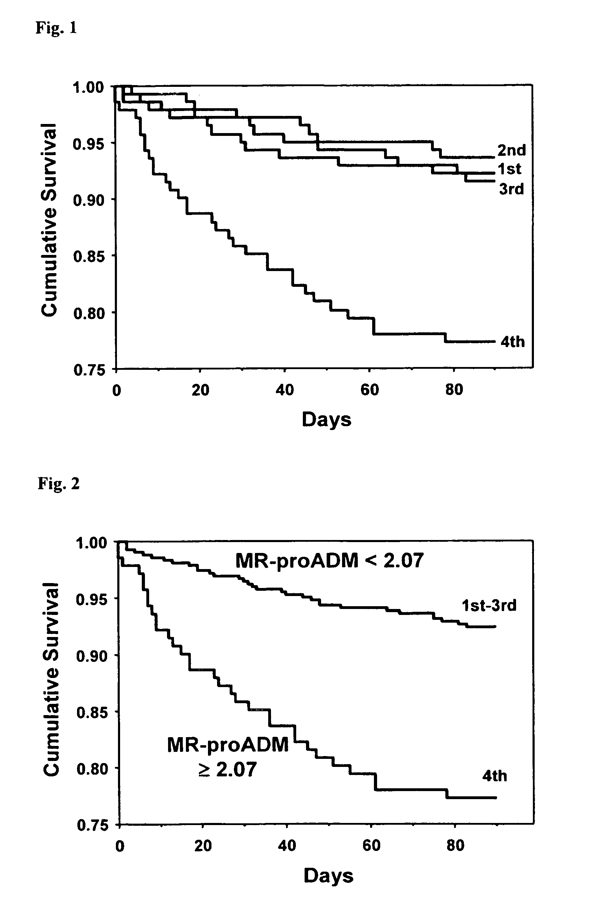

Risk is great in the highest quartile of MR-proADM, see Fig. 1 and 2 and tables 9 and 10.

**Table 9:**

| **Quartile** | **HR** | **95% CI** | **p** |
|---|---|---|---|
| 1st | 1 | reference | |
| 2nd | 0.8 | 0.3-2.0 | 0.640 |
| 3rd | 1.1 | 0.5-2.5 | 0.822 |
| 4th | 3.2 | 1.6-6.4 | 0.001 |

**Table 10:**

| **Quartile** | **HR** | **95% CI** | **p** |
|---|---|---|---|
| 1 st-3rd | 1 | reference | |
| 4th | 3.3 | 2.0-5.4 | <0.001 |

### Survival in AHF - BNP Quartiles

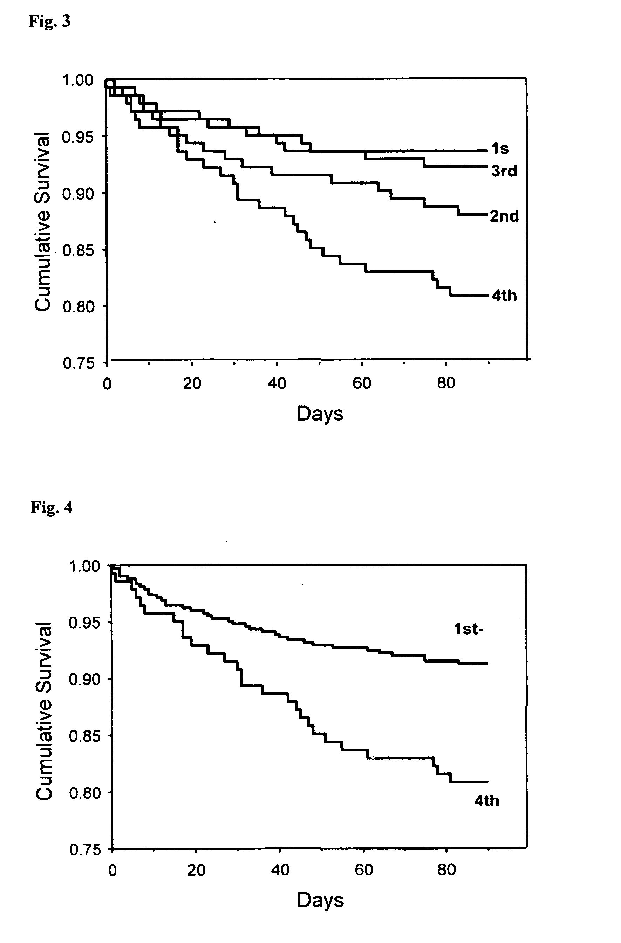

Risk is great in the highest quartile of BNP, see Fig. 3 and 4 and tables 11 and 12.

**Table 11:**

| **Quartile** | **HR** | **95% CI** | **p** |
|---|---|---|---|
| 1st | 1 | reference | |
| 2nd | 1.9 | 0.9-4.3 | 0.116 |
| 3rd | 1.2 | 0.5-2.9 | 0.668 |
| 4th | 3.2 | 1.5-6.7 | 0.003 |

**Table 12:**

| **Quartile** | **HR** | **95% CI** | **p** |
|---|---|---|---|
| 1 st-3rd | 1 | reference | |
| 4th | 2.3 | 1.4-3.8 | <0.001 |

### Survival in AHF - NT-proBNP Quartiles

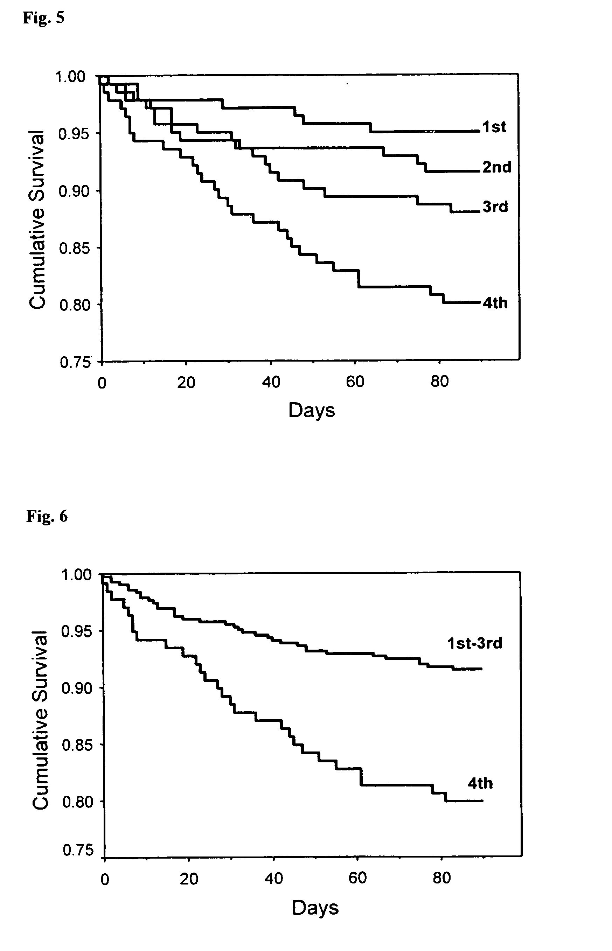

Risk is great in the highest quartile of NT-proBNP, see Fig. 5 and 6 and tables 13 and 14.

**Table 13:**

| **Quartile** | **HR** | **95% CI** | **p** |
|---|---|---|---|
| 1 st | 1 | reference | |
| 2nd | 1.7 | 0.7-4.4 | 0.247 |
| 3rd | 2.5 | 1.0-6.0 | 0.043 |
| 4th | 4.3 | 1.9-9.9 | <0.001 |

**Table 14:**

| **Quartile** | **HR** | **95% CI** | **p** |
|---|---|---|---|
| 1 st-3rd | 1 | reference | |
| 4th | 2.5 | 1.5-4.1 | <0.001 |

### Survival in AHF - Area Under the ROC Curve Comparison

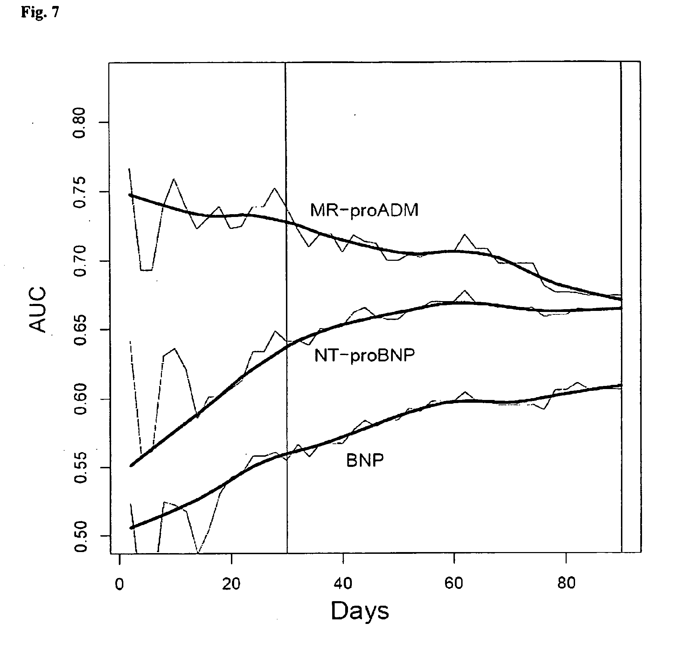

MR-proADM predicts short term (30 day) survival exceptionally well, see Fig. 7 and table 15.

**Table 15:**

| **AUC** | **30 days** | **90 days** |
|---|---|---|
| MR-proADM | 0.739 | 0.674 |
| NT-proBNP | 0.641 | 0.664 |
| BNP | 0.555 | 0.606 |

### Survival in all patients with SoB - Utility of MR-proADM

**Table 16: Cox Regression Analysis, MR-proADM performs well in all SoB patients.**

| **Predictor (univariate)** | **Chi² Statistic** | **p** | **c index** |
|---|---|---|---|
| log MR-proADM | 129.5 | <0.0001 | 0.755 |
| log BNP | 60.1 | <0.0001 | 0.691 |
| log NT-proBNP | 83.7 | <0.0001 | 0.721 |

**Table 17: Cox Regression Analysis, MR-proADM is superior to BNP and NT-proBNP.**

| | **Chi² Statistic** | **p** |
|---|---|---|
| adding MR-proADM to BNP | 69.4 | <0.0001 |
| adding MR-proADM to NT-proBNP | 46.6 | <0.0001 |
| adding BNP to MR-proADM | 0.1 | 0.731 |
| adding NT-proBNP to MR-proADM | 1.5 | 0.229 |

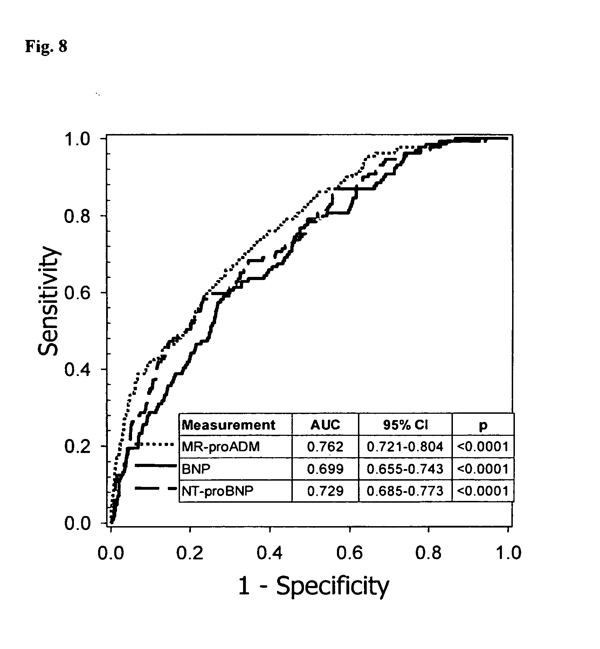

A corresponding ROC plot is shown in Fig. 8.

### Survival in patients without AHF - Utility of MR-proADM

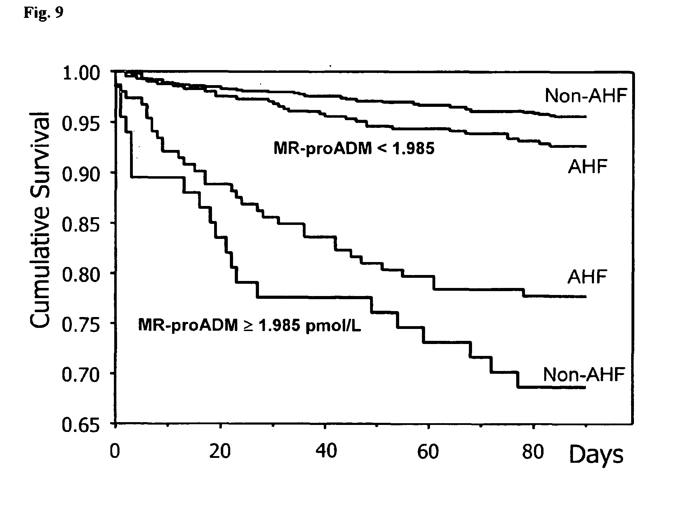

Elevated MR-proADM is strongly prognostic in patients with and without AHF - even more so in non-AHF than in AHF (interaction p=0.005). See appended Fig. 9 and tables 18 and 19.

**Table 18:**

| AHF patients | | |
|---|---|---|
| | **AUC (90 days)** | **optimal cut point from ROC** |
| MR-proADM | 0.674 | 1.985 pmol/l |

**Table 19:**

| Diagnosis | MR-proADM | HR | 95% **CI** | **p** |
|---|---|---|---|---|
| Non-AHF | low < 1.985 | 1 | reference | |
| | high ≥ 1.985 | 8.6 | 5.1-14.4 | <0.001 |
| AHF | low < 1.985 | 1.7 | 1.1-2.7 | 0.027 |
| | high ≥ 1.985 | 5.7 | 3.6-8.9 | <0.001 |

### Summary of study:

- MR-proADM is a strong prognosticator in patients with AHF and in patients presenting with SoB.
- MR-proADM is superior to BNP or NT-proBNP for predicting 90-day mortality, both in AHF as well as in all ED pts with SoB.
- MR-proADM is particularly strong in predicting short-term prognosis within 4 weeks after assessment.
- All these results are unaffected by adjustment for Troponin.
- MR-proADM can significantly improve risk stratification over BNP or NT-proBNP.
- Assessment of MR-proADM can help to identify patients who should "move to the front of the line" of medical care.

## Claims

1. A method for prognosis of an outcome after a time period of up to 90 days of a patient suffering from heart failure and/or shortness of breath, comprising the steps of:
a. providing a sample obtained from said patient,
b. determining the level of adrenomedullin (ADM) or fragments thereof or its precursor or fragments thereof in said sample, wherein the fragments are of at least six amino acids in length
c. correlating said level with said prognosis.

2. A method according to claim 1, wherein the level of troponin is determined in addition to ADM or fragments thereof or its precursor or fragments thereof.

3. A method according to claim 1 or 2, wherein said outcome regards survival and/or a functional outcome.

4. A method according to claims 1 to 3, wherein the outcome after 3 days, 5 days, 10 days, 14 days, 20 days, 3 weeks, 4 weeks, 30 days, preferably 30 days is predicted.

5. A method for the stratification of a patient into risk groups wherein said patient is suffering from heart failure and/or shortness of breath and said method comprising the steps according to claim 1.

6. A method according to claims 1 to 5, wherein adrenomedullin (ADM) or fragments thereof or its precursor or fragments thereof is MR-proADM.

7. A method according to claims 1 to 6, wherein said outcome regards survival.

8. A method according to claim 1 to 6, wherein the functional outcome is determined as ranking or the degree of severity of the outcome.

9. A method according to claims 1 to 8, wherein additionally at least one clinical parameter is determined selected from the group comprising: age, gender, systolic blood pressure, diastolic blood pressure, antihypertensive treatment, body mass index, heart rate, temperature, presence of diabetes mellitus, current smoking habits.

10. Use of the method according to any one of the claims 1 to 9 for monitoring of the therapy in a patient suffering from heart failure and/or shortness of breath.

## Patentansprüche

1. Verfahren für die Prognose eines Ergebnisses nach einem Zeitraum von bis zu 90 Tagen bei einem Patienten, der an Herzinsuffizienz und/oder Atemnot leidet, welches die folgenden Schritte umfasst:
a. Bereitstellen einer Probe, die von besagtem Patienten entnommen wurde,
b. Bestimmung des Adrenomedullin (ADM)-Spiegels oder des Spiegels von Fragmenten davon oder von dessen Vorstufe oder Fragmenten davon in dieser Probe, wobei die Fragmente mindestens aus sechs Aminosäuren bestehen müssen,
c. Korrelieren des besagten Spiegels mit der besagten Prognose.

2. Verfahren nach Anspruch 1, wobei zusätzlich zum ADM-Spiegel oder von Fragmenten davon oder von dessen Vorstufe oder Fragmenten davon der Troponinspiegel bestimmt wird.

3. Verfahren nach den Ansprüchen 1 oder 2, wobei die besagten Ergebnisse die Überlebensrate und/oder funktionelle Ergebnisse berücksichtigen.

4. Verfahren nach den Ansprüchen 1 bis 3, wobei das Ergebnis nach 3 Tagen, 5 Tagen, 10 Tagen, 14 Tagen, 20 Tagen, 3 Wochen, 4 Wochen, 30 Tagen, vorzugsweise nach 30 Tagen prognostiziert wird.

5. Verfahren für die Einordnung eines Patienten in Risikogruppen, wobei der besagte Patient an Herzinsuffizienz und/oder Atemnot leidet und das besagte Verfahren die Schritte gemäß Anspruch 1 umfasst.

6. Verfahren nach den Ansprüchen 1 bis 5, wobei Adrenomedullin (ADM) oder Teile davon oder dessen Vorstufe oder Teile davon MR-proADM sind.

7. Verfahren nach den Ansprüchen 1 bis 6, wobei das Ergebnis die Überlebensrate berücksichtigt.

8. Verfahren nach den Ansprüchen 1 bis 6, wobei das funktionelle Ergebnis als Rangfolge oder als Schweregrad des Ergebnisses bestimmt wird.

9. Verfahren nach den Ansprüchen 1 bis 8, wobei zusätzlich mindestens ein klinischer Parameter bestimmt wird, der aus der folgenden Gruppe ausgewählt wird: Alter, Geschlecht, systolischer Blutdruck, diastolischer Blutdruck, Behandlung gegen Bluthochdruck, Body-Mass-Index, Herzfrequenz, Temperatur, Diabetes mellitus, aktuelle Rauchgewohnheiten.

10. Einsatz des Verfahrens nach einem der Ansprüche 1 bis 9 zur Überwachung der Therapie eines Patienten, der an Herzinsuffizienz und/oder Atemnot leidet.

## Revendications

1. Procédé de pronostic d'un résultat après une période allant jusqu'à 90 jours chez un patient souffrant d'une insuffisance cardiaque et/ou d'une dyspnée, consistant à :
a. fournir un échantillon prélevé dudit patient ;
b. déterminer le niveau d'adrénomédulline (ADM) ou de fragments de celle-ci ou de son précurseur ou de fragments de celui-ci dans ledit échantillon, les fragments ayant une longueur d'au moins six amino-acides ;
c. corréler ledit niveau avec ledit pronostic.

2. Procédé selon la revendication 1, le niveau de troponine étant déterminé en plus de celui de l'ADM ou de fragments de celle-ci ou de son précurseur ou de fragments de celui-ci.

3. Procédé selon la revendication 1 ou 2, ledit résultat concernant la survie et/ou un résultat fonctionnel.

4. Procédé selon les revendications 1 à 3, le résultat étant prédit après 3, 5, 10, 14 ou 20 jours, 3 ou 4 semaines ou 30 jours, de préférence 30 jours.

5. Procédé de stratification d'un patient en groupes à risque, le patient souffrant d'une insuffisance cardiaque et/ou d'une dyspnée et ledit procédé comprenant les étapes selon la revendication 1.

6. Procédé selon les revendications 1 à 6, l'adrénomédulline (ADM) ou des fragments de celle-ci ou son précurseur ou des fragments de celui-ci étant MR-proADM.

7. Procédé selon les revendications 1 à 6, ledit résultat concernant la survie.

8. Procédé selon les revendications 1 à 6, le résultat fonctionnel étant déterminé en tant que classement ou niveau de gravité du résultat.

9. Procédé selon les revendications 1 à 8, en outre au moins un paramètre clinique étant déterminé sélectionné [sic] du groupe comprenant : l'âge, le sexe, la tension artérielle systolique, le traitement antihypertenseur, l'indice de masse corporelle, la fréquence cardiaque, la température, la présence du diabète sucré et les habitudes tabagiques actuelles.

10. Procédé selon l'une quelconque des revendications 1 à 9 pour surveiller le traitement d'un patient souffrant d'une insuffisance cardiaque et/ou d'une dyspnée.
